# FASCICULE DE BREVET EUROPEEN

(11) **EP 0 344 024 B1**
(45) Date de publication et mention de la délivrance du brevet: **14.08.1996**
(21) Numéro de dépôt: 89400768.1
(22) Date de dépôt: 17.03.1989
(51) Int. Cl.: C12N 15/81, C12N 1/19, C12N 1/21, G01N 33/50, G01N 33/566, G01N 33/74

(54) **Vecteur pour l'expression des récepteurs membranaires de mammifère dans des organismes unicellulaires et procédé d'étude de ligands reconnaissant des récepteurs**
Vektor zur Expression von Membranrezeptoren aus Säugetieren in einzelligen Organismen und Methode zur Untersuchung von Liganden, die diese Rezeptoren erkennen
Vector for the expression of mammalian membrane receptors in unicellular organisms and method of studying ligands recognising these receptors

(30) Priorité: 21.03.1988 FR 8803475
(43) Date de publication de la demande: 29.11.1989
(73) Titulaire: CENTRE NATIONAL DE LA RECHERCHE SCIENTIFIQUE, 75007 Paris (FR)
(72) Inventeur: Marullo, Stefano, F-75013 Paris (FR); Delavier, Colette, F-75014 Paris (FR); Emorine, Laurent, F-75013 Paris (FR); Strosberg, Donny, F-75015 Paris (FR)
(74) Mandataire: Gutmann, Ernest

(56) Documents cités:
- EP-A- 0 244 221
- FEDERATION PROCEEDINGS, vol. 46, no. 6, 01 mai 1987, American Society of Biological Chemists, 78th Annual Meeting, Philadelphia, PA, 7-11 juin 1987; S.T. GEORGE et al., p. 2193, no. 1562
- PROCEEDINGS OF THE NATL. ACADEMY OF SCIENCES USA, vol. 84, no. 22, novembre 1987, Washington, DC (US); T. FRIELLE et al., pp. 7920-7924
- SCIENCE, vol. 238, 30 octobre 1987, Washington, DC (US); B.K. KOBILKA, pp. 650-656
- NATURE, vol. 323, 02 octobre 1986, Londres (GB); T. KUBO et al., pp. 411-416
- PROCEEDINGS OF THE NATL. ACADEMY OF SCIENCES USA, vol. 84, octobre 1987, Washington, DC (US); L.J. EMORINE et al., pp. 6995-6999

## Description

L'invention concerne un vecteur réplicable dans une culture de levures, ce vecteur contenant un gène codant pour une protéine de mammifère et ayant une activité biologique de récepteur membranaire et interagissant avec une protéine de régulation -dite protéine G- apte à fixer des molécules de guanosine triphosphate (GTP).

L'invention est également relative à des levures transformées par des gènes codant pour ces récepteurs membranaires et portant à leur surface des séquences peptidiques reconnues par des ligands spécifiques desdits récepteurs membranaires.

L'invention concerne aussi des trousses (ou kits) pour l'identification de protéines de mammifères ayant des caractéristiques permettant de vérifier qu'ils appartiennent à une famille de récepteurs membranaires de mammifères ou, à l'inverse, d'étudier le degré d'affinité de certaines substances pour des récepteurs membranaires déterminés.

D'une façon générale, les protéines G sont des protéines ayant la capacité de s'interposer structuralement et fonctionnellement entre des récepteurs et des enzymes catalysant la production de médiateurs intracellulaires (tels que l'adénylate cyclase, la guanylate cyclase, les phospholipases, les kinases) ou entre des récepteurs et des canaux ioniques dont l'ouverture contrôlée entraîne un flux d'ions (tels que les ions calcium, potassium, sodium, hydrogène) dans la cellule.

Ces protéines ont des fonctions de transduction et de couplage et sont caractérisées par une structure monomère composée de trois sous-unités protéiques alpha, bêta, gamma. On peut distinguer différentes protéines G à partir des variations observées dans leur sous-unité alpha ou parfois dans leur sous-unité bêta. Ces protéines G ont été décrites en détail dans l'article de Allen M.Spiegel (21).

Des récepteurs membranaires de cellules de mammifères normalement couplés à une protéine G, ont été identifiés par clonage, complétés pour certains d'entre eux par l'analyse de leur séquence. Ces récepteurs comportent des régions exposées à la surface des cellules hôtes, ayant des affinités spécifiques respectives pour une grande variété de ligands, hormones, neurotransmetteurs et autres stimulants sensoriels capables de transmettre des signaux à l'intérieur desdites cellules, via les récepteurs correspondants et les protéines G.

Des récepteurs intéressants dans le cadre de l'invention sont notamment les récepteurs suivants :
- les récepteurs β adrénergiques : (récepteur aviaire β1, de mammifère β1 et β2), récepteur du Glucagon, de l'hormone adrénocorticotrope, de l'hormone parathyroide (PTH : abréviation anglaise de "Para Thyroïd Hormone"), de l'hormone thyréotrope, du facteur libérant de l'hormone de croissance, de la prostaglandine E, les récepteurs D1 dopaminergique, du peptide vasoactif intestinal, les récepteurs muscariniques M1 à M6, le récepteur de la serotonine S1,S2, le récepteur D2 dopaminergique, du neuropeptide substance K, de la vasopressine, de la somatostatine, de la thyréostimuline, de la bombésine, de la cholécystokinine (CCK), les récepteurs de la lumière, du peptide Met-Leu-Phe chemtactique, les récepteurs α adrénergiques (α1,α2), le récepteur H1 histaminique, le récepteur des opioides (enkephalines, endorphines), du neuropeptide substance P, de l'angiotensine II, de la prostaglandine, du facteur libérant l'hormone corticotrope, de la bradykinine, de la sécrétine, le récepteur olfactif.

Une famille préférée de récepteurs selon l'invention, qui présentent des caractéristiques génétiques et moléculaires qui leur sont essentiellement communes, comprend des récepteurs β1-, β2-, α1 et α2-adrénergiques (1)(2)(3)(4), des récepteurs muscariniques appartenant aux sous-types M1 à M4 (5)(6) et le récepteur du neuropeptide connu sous la désignation "substance K"(7). Ces récepteurs paraissent posséder une organisation structurale commune au sein de la membrane plasmatique, normalement caractérisée par sept segments transmembranaires hydrophobes entre lesquels s'intercalent des boucles extra- et intra-cellulaires, par une région aminoterminale extracellulaire et par une région carboxyterminale cytoplasmique.

Plusieurs gènes codant respectivement pour certains de ces récepteurs ont été rendus capables, après leur introduction dans des cultures de cellules de mammifères appartenant à des espèces étrangères de s'y exprimer. Les opérations de transformations nécessaires à l'expression de ces gènes dans des cellules de mammifères appartenant à des espèces étrangères sont assez complexes et ne peuvent être aisément généralisées à l'expression d'autres récepteurs.

L'invention a pour but de fournir des procédés et moyens rendant aisée l'identification et l'étude de nouveaux récepteurs membranaires présentant des caractéristiques structurales communes avec les récepteurs membranaires du type sus-indiqué. A l'inverse elle a pour but l'étude aisée du niveau d'affinité éventuel de molécules susceptibles de se comporter comme des ligands à l'égard de ces récepteurs membranaires.

En particulier, l'invention concerne un vecteur réplicable dans une culture de levures contenant un insérat de nucléotides codant pour une séquence d'acides aminés contenue dans une protéine de mammifère et ayant une activité biologique de récepteur membranaire, ce vecteur étant caractérisé en ce que l'insérat est placé sous le contrôle d'une séquence, incluse dans ce vecteur, autorisant l'expression de cet insérat dans ledit hôte cellulaire, et comportant notamment un promoteur reconnu par les polymérases de cet hôte unicellulaire, et en ce que cet insérat code pour une séquence d'acides aminés contenue dans une protéine présentant des éléments de structure en commun avec les récepteurs membranaires susdits, notamment les récepteurs α- ou β-adrénergiques, les récepteurs muscariniques, les récepteurs neuropeptidiques, en particulier la "substance K", l'expression de l'insérat s'accompagnant également de l'exposition à l'extérieur de ces levures des sites caractéristiques des récepteurs membranaires qui affleurent à la surface des cellules de mammifères d'origine.

L'invention résulte de la découverte que le fait de placer une séquence de nucléotides codant pour un récepteur membranaire de mammifère sous le contrôle d'un promoteur reconnu par les polymérases des levures dans des conditions permettant l'expression de cette séquence de nucléotides au sein des levures permet également, lorsque se manifeste cette expression, le transport du produit d'expression dans les membranes des levures et que cette expression s'accompagne également de l'exposition, également à l'extérieur de ces levures des sites caractéristiques des récepteurs membranaires qui affleurent à la surface des cellules de mammifères d'origine, et ce sous une configuration spatiale conservée à l'échelle moléculaire, de sorte que ces sites peuvent être reconnus par les mêmes ligands.

Par "hôte unicellulaire" on entend tout organisme susceptible d'être maintenu en culture en l'absence de différentiation cellulaire. L'organisme unicellulaire de choix est constitué par Escherichia Coli. Mais d'autres microorganismes peuvent être utilisés tout aussi aisément, naturellement sous réserve que l'on connaisse pour chacun d'entre eux des vecteurs notamment plasmidiques susceptibles de s'y répliquer et des séquences de nucléotides insérables dans ces vecteurs et capables, lorsqu'elles sont suivies dans ces vecteurs par un insérat codant pour un polypeptide ayant les propriétés d'un récepteur membranaire dans les conditions sus-indiquées, d'assurer l'expression de cet insérat dans les organismes choisis et leur transport dans les membranes de ces hôtes unicellulaires.

S'agissant de réaliser l'expression dans E.coli, la séquence dirigeant l'expression de l'insérat peut comporter, par exemple, les éléments de régulation, notamment le promoteur de l'opéron lactose ou de l'opéron tryptophane.

Des levures susceptibles d'être mises en oeuvre sont indiquées dans le tableau ci-après :

| Organisme unicellulaire | Type vecteur | Région promotrice |
|---|---|---|
| S.Cerevisiae | plasmide 2µ | alcool deshydrogénase I (ADH.I) |
| S.Cerevisiae | plasmide PBM 258 | promoteur du gène galactokinase (GAL I) |

Les organismes sus-indiqués et les vecteurs modifiés correspondants se prêtent avec avantage à l'expression de séquences génétiques codant pour des molécules ayant des caractéristiques de structure en commun avec des récepteurs membranaires déjà connus de mammifères et à leur transport à la membrane de ces hôtes unicellulaires dans les conditions sus-indiquées.

A titre d'exemples de récepteurs susceptibles d'être aussi placés et exprimés dans des organismes unicellulaires, notamment E.coli, on mentionnera :
- les différents récepteurs β-adrénergiques et muscariniques décrits dans le chapitre intitulé "the β-adrenergic receptors and G-proteins" (récepteurs β-adrénergiques et G-protéines) de A.D.Strosberg, dans l'ouvrage édité sous la direction du même auteur sous le titre "The Molecular Biology of receptors - Techniques and Applications of Receptor Research" (La Biologie Moléculaire des récepteurs-Techniques et Applications de la recherche de récepteurs) et publié par Ellis Horwood Ltd. Chichester - Grande Bretagne (VCH)
- ou encore les récepteurs décrits dans les publications de Brian K.Kobilka et al, Nature, Vol. 329, 75-79 du 3 Septembre 1987 ou de L.J.Emorine et al. Proc. Natl. Acad. Sci. USA, vol. 84, p. 6995-6999, Octobre 1987.

L'invention permet non seulement l'étude des récepteurs membranaires de mammifères qui ont été indiqués, mais aussi de ceux des récepteurs ou polypeptides ayant des éléments de structures semblables, qui peuvent être obtenus ou purifiés à partir de techniques classiques, par exemple
- soit par des cADNs obtenus par transcription reverse d'ARN messagers et séquencés, par exemple selon les techniques décrits par Dixon.R.A.F. et al (1986) Nature, 321, 75, Dixon,R.A.F. et al (1987)Nature, 326, 73, Yarden, Y. et al (1987) Proc. Natl. Acad. Sci. USA 83, 6795-6799,
- soit en ayant recours à des oligonucléotides utilisés comme sondes d'hybridation, permettant l'isolement de polynucléotides codant pour des récepteurs du gène en question selon des techniques connues, comprenant des réactions d'hybridation sélective.

Lesdites sondes consistent par exemple en des oligonucléotides-sondes dérivés de fragments d'ADN de régions à fortes homologies entre les différents récepteurs.

Des sondes utilisables pour isoler des séquences de nucléotides codant pour des récepteurs membranaires peuvent aussi, par exemple, consister en des fragments conservés, obtenus à partir de séquences nucléotidiques codant pour l'une des sept séquences peptidiques transmembranaires hydrophobes représentées aux pages 148-149 de l'ouvrage déjà cité et édité sous la direction de A.D.Strosberg. Ces différentes séquences peptidiques respectivement visées par les chiffres romains de I à VII, sensiblement analogues entre-elles sont issues respectivement de récepteurs β2-adrénergiques de hamster, de récepteurs β-adrénergiques de l'homme, de récepteurs β-adrénergiques de la dinde, de récepteurs muscariniques de l'acétyl choline de cerveau de porc et d'opsine de bovin.

Dans le cas de l'opsine de bovin, on aura recours, pour préparer une des sondes précédentes, à l'ADNc correspondant à l'ARN génomique du récepteur.

A titre d'exemple, les sondes suivantes pourront être mises en oeuvre pour isoler des séquences nucléotidiques codant pour des récepteurs membranaires. CTTGGTCAGCTTGGCCTGTGCTGATCTGGTCATG (segment transmembranaire II)
GCTTTCATGGTCAGCTGGCTGCCCTAC (segment transmembranaire VI)

On peut aussi mettre en oeuvre des ensembles de sondes généralement représentables par les formules suivantes :
- ensembles transmembranaires II :
   5'X₁X₂TX₃X₄TX₅X₆X₇CX₈TGGCX₉X₁₀X₁₁X₁₂GCTGAX₁₃CTX₁₄X₁₅ TX₁₆ATG3'
   dans laquelle X₁ à X₁₆ ont indépendamment les uns des autres, les significations suivantes :
   X₁ est C ou T
   X₂ est T ou A
   X₃ est G ou C
   X₄ est G ou T
   X₅ est C ou G
   X₆ est A ou T
   X₇ est G ou C
   X₈ est T ou C
   X₉ est C ou G
   X₁₀ est T ou G
   X₁₁ est G ou T
   X₁₂ est T ou G
   X₁₃ est T ou C
   X₁₄ est G ou C
   X₁₅ est G ou T
   X₁₆ est C ou G
   et ensembles transmembranaires VI :
   5'GCY₁TTCATY₂Y₃TY₄Y₅Y₆CTGGY₇TY₈CCY₉TY₁₀C3'
   dans laquelle Y₁ à Y₁₀ représentent indépendamment les uns des autres :
   Y₁ est T ou G
   Y₂ est G ou C
   Y₃ est G ou C
   Y₄ est C ou G
   Y₅ est A ou T
   Y₆ est G ou C
   Y₇ est C ou G
   Y₈ est G ou C
   Y₉ est C ou G
   Y₁₀ est A ou T

Pour isoler des séquences nucléotidiques présumées coder pour un récepteur membranaire, on utilisera de préférence deux ou plusieurs séquences sondes telles que définies ci-dessus, choisies pour leur appartenance à des régions différentes des séquences nucléotidiques présentant une communauté de séquence dans la partie correspondant aux sept segments transmembranaires décrits plus haut. En particulier, on mettra on oeuvre des sondes du type de celles décrites plus haut avec leurs séquences complémentaires respectives.

On pourra également avoir recours à des ensembles des ces sondes.

On apprécie que le procédé selon l'invention permet alors, pour chaque récepteur membranaire exprimé à la surface des organismes unicellulaires, à l'exception des lignées cellulaires de mammifères, de mesurer les degrés d'affinité de différentes molécules présumées se comporter comme ligands vis-à-vis de ces récepteurs. Ces ligands peuvent d'ailleurs être utilisés également pour repérer au sein de colonies diverses transformées un grand nombre de fragments d'acide nucléique d'origines diverses (expérience dite de "shot gun") parmi lesquels des fragments codant pour ces polypeptides de mammifères. Seront alors détectées celles des colonies qui, en raison de l'expression d'une séquence codant pour le récepteur membranaire de mammifère, forment une liaison avec le ligand correspondant.

A l'inverse, l'invention permet aussi l'étude de l'affinité de diverses molécules possibles à l'égard d'un récepteur membranaire déterminé consistant dans le produit d'expression d''un insérat correspondant inséré dans un vecteur modifié dans les conditions sus-définies et incorporé à un hôte cellulaire dans lequel ce vecteur est réplicable.

De même l'invention permet de comparer les affinités réciproques d'un récepteur déterminé avec différentes molécules ayant des motifs structuraux en commun avec des récepteurs différents membranaires de mammifères, d'où la possibilité de classer lesdits récepteurs par ordre d'affinité. Tout ligand approprié peut être mis en oeuvre dans ces essais comparatifs. On mentionnera à titre d'exemple de ligands le pindolol, l'iodocyano-pindolol, le propranolol, l'alprenolols, etc...On peut encore se référer à l'ouvrage édité sous la direction de A.D. Strosberg, dans lequel sont identifiés de nombreux autres agonistes, ou antagonistes de récepteurs membranaires répondant aux définitions de l'invention.

Dans l'une de ses applications préférées l'invention concerne donc des cultures de levures ou de bactéries, contenant des vecteurs réplicables au sein de ces organismes, ces vecteurs répondant aux conditions indiquées plus haut et contenant un insérat codant pour un récepteur membranaire de mammifères répondant aux définitions sus-indiquées, l'expression de ces insérats étant inductible ou ayant fait l'objet d'une induction entrainant le transport à la membrane des récepteurs membranaires correspondants, selon la nature du promoteur utilisé.

Dans un mode de mise en oeuvre préféré de l'invention, le vecteur modifié est caractérisé en ce que l'insérat code pour une séquence d'acides aminés contenue dans un récepteur de mammifère du genre sus-indiqué, comportant dans sa structure des segments transmembranaires hydrophobes intercalés dans des boucles extra et intra-cellulaires, un segment intra-cellulaire carboxy-terminal, et un segment amino-terminal extracellulaire et des sites de liaisons constitués par des régions hydrophiles formées dans la membrane.

Pour la réalisation du vecteur selon l'invention, on mettra en oeuvre de manière avantageuse un promoteur inductible. Un tel promoteur est régulable et n'est fonctionnel qu'après induction par une molécule biochimique ou par un traitement physique (traitement par la température, les rayons ultra violets...). L'utilisation d'un tel promoteur inductible permet donc de faire exprimer la séquence nucléotidique contenue dans le vecteur d'expression modifié, dans des conditions préalablement déterminées par l'opérateur, seulement au moment de l'emploi. De cette façon on évite la dégradation des cellules de la culture hôte transformée par le vecteur modifié, chaque fois que le récepteur étranger devant être exprimé dans la cellule et transporté dans la membrane présente un certain niveau de toxicité pour cette culture.

Suivant un autre mode de réalisation du vecteur selon l'invention, l'insérat codant pour le récepteur peut être placé en aval d'une "séquence leader" ou "séquence signal" et en phase avec celle-ci, pour former une unité de transcription, cette séquence "leader" devant alors faciliter le transport du produit d'expression de l'insérat jusqu'à la membrane de l'hôte unicellulaire. Cette séquence "leader" code normalement pour une séquence N-terminale d'un précurseur d'une protéine de l'hôte unicellulaire, normalement transportée à la membrane de la cellule, ce peptide N-terminal étant normalement clivé à l'occasion du transport de la protéine naturelle correspondante à la membrane de l'hôte unicellulaire. De la même façon le gène de fusion entre cette séquence "leader" ou signal et l'insérat doit normalement être exprimé sous la forme d'une protéine hybride clivée en aval de la séquence signal à l'occasion du transport du récepteur à la membrane de l'hôte unicellulaire.

En variante, l'insérat est fusionné à une séquence codant pour tout ou partie d'une protéine hydrophile (par exemple la β-galactosidase) cette fusion permettant d'assurer la solubilité de la protéine hybride, produit de l'expression de l'insérat et de la séquence codant pour la protéine hydrophile, pendant le transport du récepteur résultant de l'expression de l'insérat dans l'hôte unicellulaire et dans la membrane de celui-ci.

L'invention se rapporte encore aux levures elles-mêmes transformées par un vecteur modifié tel que décrit plus haut, et plus particulièrement des cultures de levures portant à leur surface des sites ou déterminants reconnus par les ligands spécifiques des récepteurs membranaires de mammifères correspondants.

Pour mettre en oeuvre l'invention et réaliser l'expression de la séquence nucléotidique correspondant à la protéine choisie, on utilisera les bactéries ou les levures.

L'invention est également relative à un procédé pour l'obtention de levures ou de bactéries comportant, exposées à leur surface, des sites caractéristiques d'un récepteur membranaire de mammifère, ce procédé étant caractérisé en ce qu'il comprend :
a/ - la transformation de levures ou de bactéries, par un vecteur réplicable dans cet organisme, préalablement modifiées contenant un promoteur reconnu par les polymérases de ces levures ou bactéries et fonctionnel dans celui-ci et un insérat placé sous le contrôle de ce promoteur et codant pour une séquence d'acides aminés normalement contenue dans un récepteur membranaire de mammifère tel qu'il a été décrit plus haut.
b/ - la sélection des levures ou bactéries transformées, dans lesquelles le susdit insérat est exprimé ou exprimable sous une forme telle que sa structure comprenne un ou plusieurs sites, exposés à l'extérieur des membranes cellulaires et capable(s) d'être reconnu par un ligand correspondant. En variante la détection de l'expression peut être réalisée en ayant recours à des anticorps spécifiques, notamment monoclonaux, reconnaissant spécifiquement les mêmes sites.

Lorsque les levures sont transformées par un vecteur modifié contenant un promoteur inductible en amont de la séquence nucléotidique à exprimer, une étape intermédiaire entre les étapes a/ et b/ précitées est nécessaire, cet étape consistant en l'induction dudit promoteur, notamment sous l'effet de la substance appropriée (par exemple l'IPTG (isopropyl-D-thiogalactopyranoside) dans le cas du promoteur de l'opéron lactose par le tryptophane dans le cas du promoteur de l'opéron tryptophane) ajoutée au milieu de culture lorsque cette induction est souhaitée.

La présente invention est encore relative à un procédé de détection de la capacité d'une molécule à se comporter comme un ligand vis-à-vis d'un récepteur membranaire, ce procédé comprenant :
a/ - la mise en contact de la molécule avec un organisme unicellulaire à l'exception des lignées cellulaires de mammifères préalablement transformé par un vecteur lui-même modifié par un insérat codant pour ce récepteur membranaire, cet organisme portant à sa surface un ou plusieurs sites spécifiques de ce récepteur, le cas échéant après induction de l'expression de cet insérat, cette mise en contact étant effectuée dans des conditions permettant la formation d'une liaison entre l'un au moins de ces sites spécifiques et ladite molécule dès lors qu'elle s'avèrerait effectivement posséder une affinité pour ce récepteur.
b/ - la détection de la formation éventuelle d'un complexe du type ligand-récepteur.

Les conditions permettant la formation de la susdite liaison, correspondent normalement à celles dans lesquelles celles-ci se forment entre les récepteurs naturels et les ligands concernés.

L'invention est aussi relative à un procédé pour l'étude de l'affinité d'un récepteur de mammifère appartenant à la classe de ceux qui sont couplés à une protéine G, pour un ou plusieurs ligands déterminés, caractérisé par :
- la transformation d'une culture unicellulaire à l'exception des lignées cellulaires de mammifères avec un vecteur, notamment un plasmide ou un phage dans lequel avait auparavant été incorporée une séquence de nucléotides ou codant pour le polypeptide contenu dans ce récepteur de mammifère, sous le contrôle d'éléments de régulation, notamment d'un promoteur, permettant l'expression dans la culture unicellulaire utilisée de ladite séquence de nucléotides,
- la culture des organismes unicellulaires transformés dans des conditions permettant l'expression dudit insérat,
- la mise en contact de ces organismes unicellulaires avec ces ligands déterminés,
- la détection d'une réaction affine entre lesdits organismes unicellulaires transformés et lesdits ligands déterminés.

L'invention vise également une trousse (ou kit) pour la détection de l'affinité éventuelle d'un ligand pour un récepteur membranaire de mammifère déterminé, comprenant :
- une culture de levures transformées par un vecteur modifié tel que décrit dans les pages précédentes, dans lequel l'insérat code pour le susdit récepteur membranaire,
- des moyens physiques ou chimiques pour induire l'expression de la séquence nucléotidique contenue dans le vecteur modifié, lorsque le promoteur placé en amont de cette séquence est un promoteur inductible par lesdits moyens physiques ou chimiques,
- un ou plusieurs ligands témoins ayant des affinités déterminées pour ledit récepteur membranaire,
- des moyens physiques ou chimiques pour la caractérisation de l'activité biologique de ladite protéine.

L'invention fournit donc des moyens permettant aussi l'étude du comportement de protéines fonctionnelles en tant que récepteurs, grâce à l'exposition de leurs sites spécifiques à la surface d'organismes unicellulaires lorsque ceux-ci auront auparavant été transformés par des vecteurs contenant un insérat codant pour ladite protéine fonctionnelle. Elle permet aussi la détection ou la vérification de l'affinité d'une protéine en tant que récepteur vis-à-vis d'un ligand déterminé. Elle permet encore l'étude de la spécificité de liaison de ces récepteurs vis-à-vis de différents ligands choisis pour leur intérêt, par exemple dans la perspective de traitements thérapeutiques futurs.

Un avantage particulier de l'invention résulte dans la substitution des récepteurs dont la purification est extrêmement difficile et la durée de conservation minime, par des cellules exprimant -ou susceptibles d'exprimer à leurs surfaces, les sites caractéristiques de récepteurs qui peuvent être reconnus par les ligands étudiés. Ces cellules peuvent être aisément conservées, surtout lorsque le vecteur modifié comprend un promoteur inductible au moment de l'emploi. Grâce à leur capacité à être cultivée, les cellules permettent la régénération à la demande des sites spécifiques de ces récepteurs en quantités pratiquement illimitées.

Les cultures d'organismes unicellulaires transformés par un vecteur modifié précédemment décrit peuvent être notamment conservées en l'attente d'usage notamment en les plaçant dans du glycérol 20% en final dans la solution à -70°C. Elles peuvent encore être conservées sous forme de spores.

L'invention se prête à de nombreuses applications. On en mentionne quelques unes ci-après à titre d'exemples.

Des applications particulièrement intéressantes en particulier dans le domaine de la thérapie consistent dans l'étude de la capacité du substances β-bloquantes nouvelles susceptibles d'agir au niveau des récepteurs β-adrénergiques, notamment β1-adrénergiques (au niveau du coeur) ou β2-adrénergiques (dans le poumon).

On a constaté que de nombreuses substances dites β-bloquantes se lient à la fois aux récepteurs β1 et β2-adrénergiques. Certains traitements thérapeutiques nécessitent cependant l'utilisation de médicaments spécifiques de l'un ou l'autre de ces récepteurs et impliquent donc la détermination de sous-types de récepteurs, capables de se lier à des ligands spécifiques. L'invention permet la réalisation de cultures unicellulaires capables de faciliter le criblage des ligands selon les natures de leurs activités vis-à-vis des différents types de récepteurs β-adrénergiques : elle permet donc la détermination aisée des spécificités pharmacologiques et thérapeutiques de médicaments à l'étude.

L'invention fournit aussi des moyens simples pour l'étude des effets de modifications introduites dans des récepteurs, vis-à-vis de leurs interactions avec des substances chimiques, notamment de ligands connus pour leur aptitude à se lier avec ces récepteurs dans leurs formes non modifiées.

D'autre caractéristiques et avantages de l'invention apparaissent dans la suite de la description et dans les exemples, notamment en rapport avec les dessins et le tableau dans lesquels :
- la figure 1 représente de façon schématique les régions codant pour le récepteur humain β2-adrénergique (hu-β2 R) recombiné avec le gène de la β-galactosidase (lac Z) du phage λgt11 (11).
- La figure 2 témoigne des variations de l'affinité de liaison entre les molécules d'un ligand, le ICYP et des cellules intactes de E.coli exprimant la séquence hu-β2R de la figure 1 contenue dans le gène de fusion.
- La figure 3 témoigne de l'expression détectée à l'aide d'un marquage par photo-affinité de récepteurs β2-adrénergiques exprimés dans la membrane de E.coli.
- Le tableau 1 contient les résultats observés avec des ligands β-adrénergiques liant le récepteur hu-β2 exprimé dans des cellules E.coli intactes et leur comportement en présence d'agonistes et d'antagonistes compétitifs.

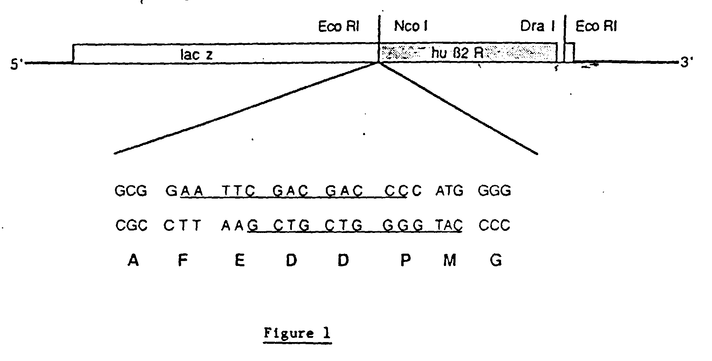

Sur la figure 1 apparaissent les sites de restriction utilisés pour la construction du gène de fusion. Le codon d'initiation pour le gène hu-β2 R est compris dans le site de restriction NcoI et son codon de terminaison est localisé 19 paires de bases en amont vers l'extrémité 5′ du site de restriction Dra I.

Dans la partie inférieure de la figure, à la jonction entre les deux gènes, l'adaptateur synthétique s'étendant des sites Eco RI à NcoI et qui maintient le cadre de lecture du gène lac Z en correspondance avec celui du gène hu-β2 R est souligné. La séquence d'acides aminés correspondante est représentée sous la séquence nucléotidique, en utilisant les codes suivants pour la désignation des aminoacides :
- M: Méthionine
- L: Leucine
- I: Isoleucine
- V: Valine
- F: Phenylalanine
- S: Sérine
- P: Proline
- T: Thréonine
- A: Alanine
- Y: Tyrosine
- H: Histidine
- Q: Glutamine
- N: Asparagine
- K: Lysine
- D: Acide Aspartique
- E: Acide glutaminique
- C: Cystéine
- W: Tryptophane
- R: Arginine
- G: Glycine

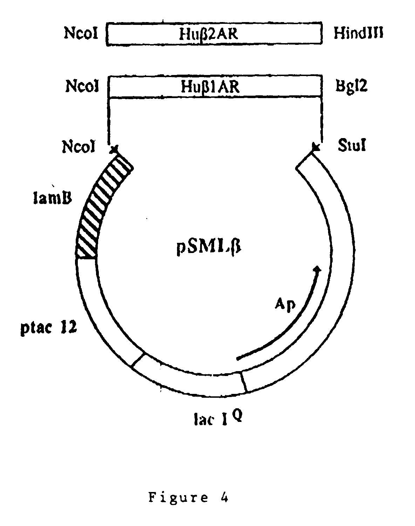

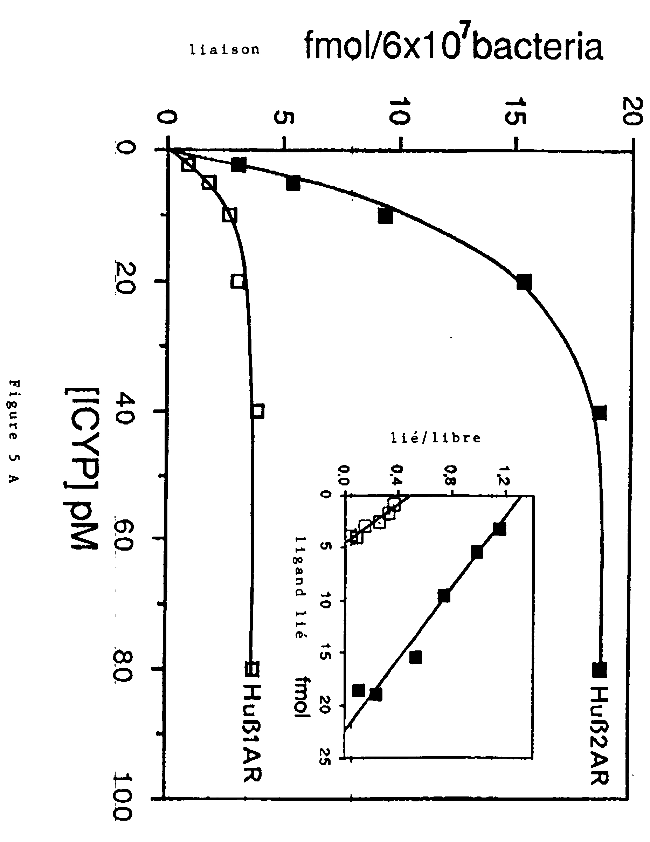

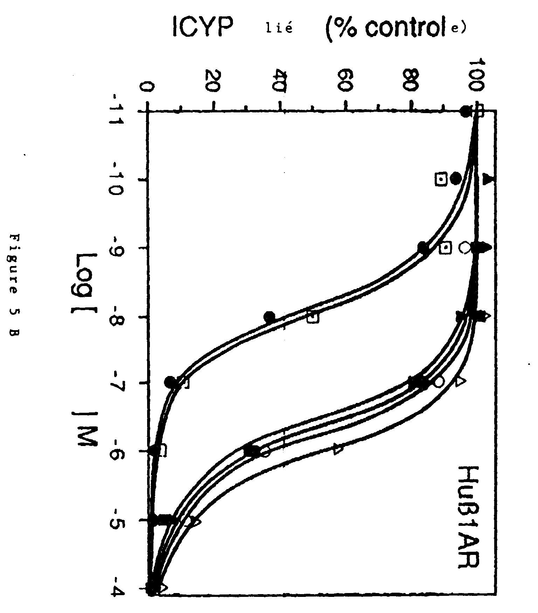

- la figure 4 représente la construction des plasmides pSMLβ1 et pSMLβ2,

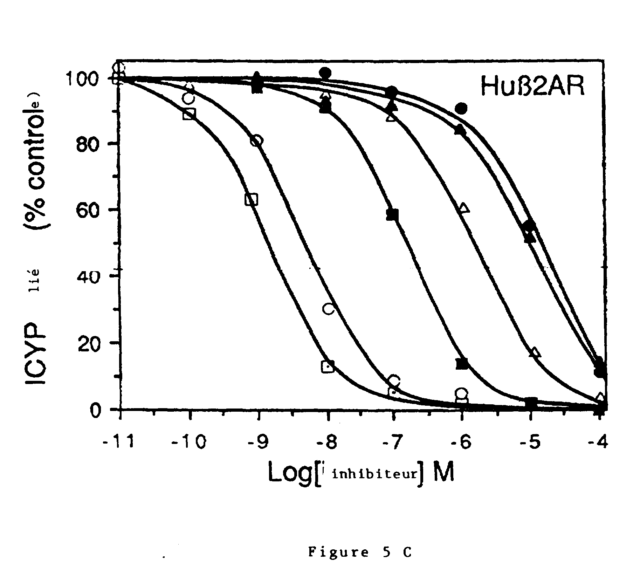

- la figure 5 représente successivement :
   A : courbes de saturation et la représentation de scatchard (voir l'encadré) de l'ICYP par rapport aux bactéries intactes exprimant Huβ1AR ou Huβ2AR. Les résultats correspondent à deux expériences réalisées sur la même culture de bactéries et sont représentatives de 4 préparations différentes. Une classe de sites de liaison est présente sur chaque récepteur. Le K_{D} calculé est 16,40 ± 0,45 pM pour Huβ1AR et 17,00 ± pM pour Huβ2AR.
   B et C : représentent des expériences d'inhibition de liaison réalisées sur Huβ1AR (B) et sur Huβ2AR (C) exprimés par E.coli intact pour déplacer 10pM de ICYP (-)Epinephrine (Δ), (-)Norepinephrine (▲), (-)propanolol (□), (+)propanolol (■), CGP 20712-A (●) ICI118,551 (○)

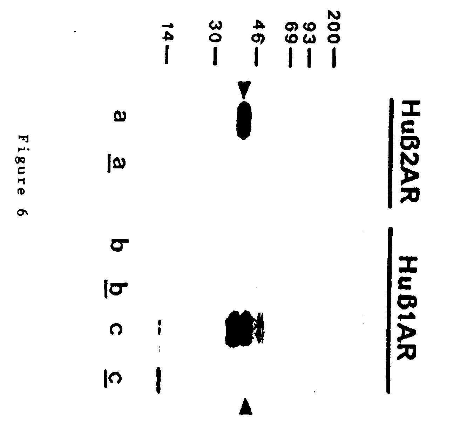

- figure 6 : cette figure représente le marquage photoaffine de Huβ1AR (lignes b et c) et Huβ2AR (lignes a) sur E.coli intact. Des expériences ont été réalisées en présence (lettres soulignées) ou en l'absence de propanolol froid de façon à montrer la liaison spécifique. Les flèches indiquent le poids moléculaire attendu des HuβARs séparés du polypeptide LamB.

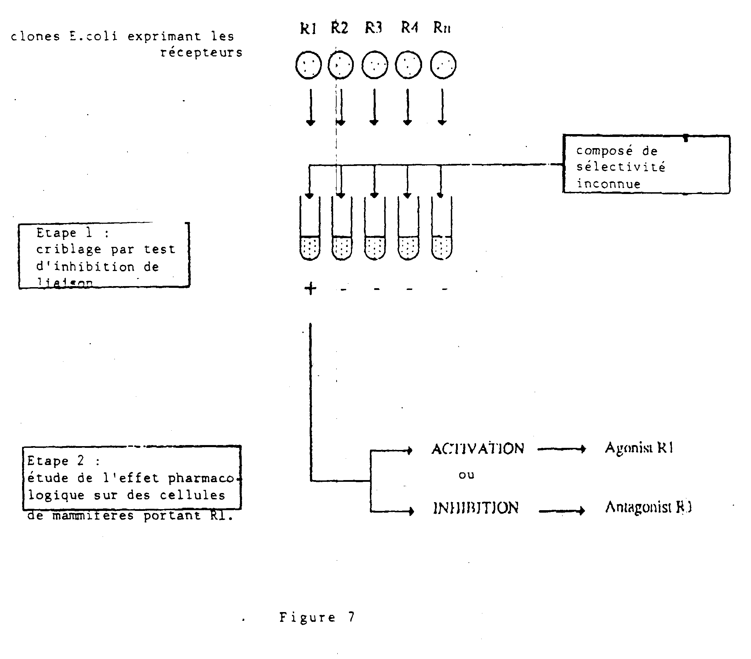

- figure 7 : la figure 7 est une représentation schématique de la procédure proposée pour le criblage de ligands interagissant avec les récepteurs β-adrénergiques.

### Exemple 1 : Expression du récepteur humain β2-adrénergique dans E.coli.

### 1/ CONSTRUCTION DU GENE RECOMBINANT.

Dans cette étape il s'agit d'insérer la région codante du récepteur humain β2-adrénergique dans le vecteur d'expression λgt11 au niveau du site de restriction Eco RI, près de l'extrémité 3′ du gène lac Z codant pour la β-galactosidase.

Dans une première étape, un adaptateur synthétique (linker) s'étendant du site Eco RI au site NcoI) est lié au fragment d'ADN purifié de 1,3kb s'étendant des sites NcoI à DraI, contenant la région codante du récepteur humain β2-adrénergique.

Cet adaptateur contient une séquence codant pour la séquence d'acides aminés Asp-Asp-Pro et facilitera la séparation de la protéine de fusion obtenue après l'étape ultérieure d'expression du gène recombinant, par hydrolyse de la liaison Asp-Pro, au moyen d'un clivage acide doux.

Après extraction au phénol et précipitation à l'isopropanol, des adaptateurs Eco RI ont été liés à cette construction qui, après digestion par Eco RI et purification, a été insérée au niveau du site unique EcoRI dans le vecteur λgt11. Un contrôle a été effectué pour vérifier que les sites de restriction Eco RI et NcoI ainsi que l'orientation du gène inséré avaient été préservés.

Le fragment de recombinaison (ou gène recombinant) obtenu entre le fragment issue du gène lac Z et le fragment hu-β2 R par l'intermédiaire de l'adaptateur sont représentés à la figure 1. Elle fait également apparaître en traits fins les parties 3′ et 5′ de l'ADN de λgt11, de part et d'autre du fragment de recombinaison.

### 2/ EXPRESSION DU GENE RECOMBINANT DANS E.coli.

Le vecteur recombinant obtenue a été inséré dans une souche Y1089 E.coli par lysogènisation de cette dernière avec le phage recombinant par une technique classique, notamment celle décrite dans (11). Les cellules ont été récoltées et remises en suspension dans un tampon 10mM tris, pH 7,4, 90mM NaCl. L'expression de la protéine de fusion peut être obtenue après induction du promoteur par un choc thermique et par la mise en cotact des cellules transformées avec l'isopropyle β-D-thio-galactopyranoside (IPTG). Une analyse du lysat bactérien par un test immunoblot en présence d'anticorps anti-β-galactosidase a montré la présence de produits dont la taille variait entre 100 et 162kDa.

### 3/ ETUDE DE L'ACTIVITE DES RECEPTEURS AINSI EXPRIMES A LA SURFACE DE E.coli, AVEC DES LIGANDS CAPABLES DE FORMER DES LIAISONS DU TYPE RECEPTEUR-LIGAND.

Pour réaliser cette étude on utilise plusieurs ligands-β-adrénergiques ainsi que des contrôles négatifs comprenant par exemple la bactérie E.coli transfectée avec le vecteur d'expression dépourvu de séquence nucléotidique codant pour le récepteur β-adrénergique, ou encore ce vecteur contenant la susdite séquence insérée dans le sens opposé au sens de lecture.

Des cellules E.coli Y1089 exprimant le gène chimérique sous forme d'une protéine de fusion, sont en suspension dans une solution de 10mM Tris (à pH 7,4), et 90mM NaCl. Des aliquots contenant 6 x 10⁷ cellules sont ensuite, après induction de l'expression du récepteur, incubés pendant 1 heure à 37°C dans un volume final de 1ml de ICYP ([¹²⁵I] iodocyanopindolol, 2080Ci/mmol, des Laboratoires AMERSHAM) à différentes concentrations (de 1 à 80pM).

Les réactions sont stoppées par filtration sur des filtres WHATMAN GF-F, suivie par 4 lavages rapides avec 4 ml de tampon. L'existence de fixations non spécifiques est déterminée en présence de 1 µM de propanolol ; ces fixations sont inférieures à 23%.

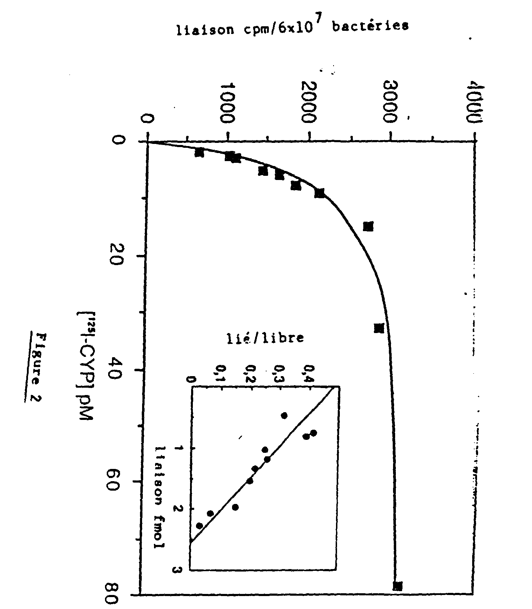

La figure 2 représente la courbe montrant la variation de la proportion de ligand fixé par 6x10⁷ bactéries, en fonction de la concentration en ligand (ICYP) (de 1 à 80 pM de [125] ICYP). Cette courbe est une courbe de saturation ; elle correspond à une classe unique de sites de fixation caractérisés par un Kd de 5,3 ±1,9 x 10⁻¹² M (r=0,93). Le calcul montre que le nombre maximum de sites permettant la liaison du récepteur au ligand est de 25,5 ± 5,2 par bactérie.

Les expériences semblables réalisées avec des bactéries infectées par le vecteur λgt11 sauvage (dépourvu du gène du récepteur humain β2) ou avec des bactéries infectées par un phage contenant le gène de ce récepteur, inséré dans la direction opposée, a montré que moins de 0,5% de liaisons a été mesuré.

On a noté que l'expression du récepteur humain β2-adrénergique est maximale après 2 heures d'induction et stable jusqu'à 20 heures. Ceci correspond à la densité de bactéries maximum ; cette observation suggère qu'il existe un effet inhibiteur sur la croissance bactérienne du produit exprimé par le gène inséré dans le vecteur d'expression.

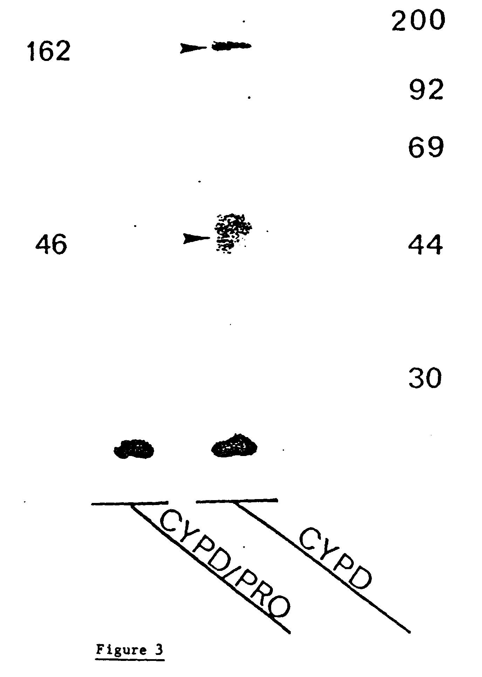

La fixation ou la liaison du ligand peut être déplacée par des ligands distincts susceptibles d'entrer en compétition avec le premier (voir par exemple le déplacement complet du ligand ICYP par le ligand hydrophile CGP 12177 dans les essais décrits plus loin en rapport avec le tableau. Après séparation du cytoplasme l'activité reste liée avec les membranes (voir discussion de la fig. 3).

Les cellules intactes non induites ont pu être conservées dans un mélange de 10mM Tris, 90mM NaCl à pH 7,4 pendant au moins 3 jours à 4°C, sans qu'il y ait perte de leur activité de liaison à l'égard du ligand.

### 4/ MARQUAGE PAR PHOTOAFFINITE DU RECEPTEUR β2-ADRENERGIQUE EXPRIME DANS LA MEMBRANE DE E.COLI

Des bactéries préparées selon un protocole analogue à celui décrit dans l'étape précédente, sont resuspendues dans un volume correspondant à 1/20 de leur volume de culture d'origine dans un tampon contenant 10 mM Tris (à pH 7,4), 150 mM NaCl, 0,5 mM de fluorure de sulfonyl-phénylméthane, 2 mM EDTA, 5µg par ml de leupeptine, 7µg par ml de pepstatine, 10.000 U par ml de trasylol et 4 µg par ml de benzamidine. Ce tampon sera encore utilisé dans toutes les étapes ultérieures.

Les cellules sont ensuite congelées (dans de l'azote liquide) puis dégelées 4 fois jusqu'à obtenir leur lyse complète. L'ADN est ensuite brisé par ultrasons (3 périodes de 10 secondes à 10 watts) pour diminuer la viscosité, et le lysat est centrifugé 20 minutes à 6.000 g pour en retirer les débris et les cellules non lysées.

Le surnageant a ensuite été centrifugé pendant 2 heures à 100 000 g. Le culot de centrifugation a été resuspendu dans 4 ml de tampon, homogénéisé dans un récipient de verre, et l'activité de liaison avec l'ICYP a été mesurée à la fois dans le culot de centrifugation et dans les fractions de surnageant.

L'activité associée avec les protéines cytoplasmiques était de 0% (déterminée par précipitation dans 12% de polyéthylène glycol 6.000) alors que 26,6% de l'activité de liaison ou fixation initiale a été retrouvée dans la fraction membranaire.

Pour le marquage en photoaffinité, environ 200fmoles de sites capables de se lier avec l'ICYP, ont été incubés dans 1ml dans l'obscurité pendant 1 heure avec 500pM de ICYP-D (Iodo-Cyano-Pendolol-Diazirine) (AMERSHAM 1800 Ci/mmol) à 37°C, en présence ou non de 5µM (-) propanolol. 4ml de tampon ont été ajoutés, et les membranes ont été lavées 3 fois par centrifugation (15 minutes à 45.000 g) et resuspendues. Une photolyse a été effectuée dans des conditions décrites précédemment (15). Les membranes marquées ont été solubilisées dans un tampon Laemmli (16) contenant 5% SDS, puis soumises à l'électrophorèse sur gel de polyacrylamide à 10% et autoradiographiées.

Ces résultats montrent que l'activité du récepteur est pour l'essentiel concentrée dans les membranes, comme en témoignent les identifications des produits de fusion repérés à 162 kDA et du récepteur libre (46 kDa).

Les données présentées dans le tableau résultent d'expériences de liaison par compétition réalisée avec des bactéries telles que celles auxquelles la figure 2 se réfère en utilisant 20 pM d'ICYP comme ligands marqués radioactivement, et différentes concentrations de ligands différents capables d'entrer en compétition avec le premier. L'analyse des effets de compétition des ligands sur les courbes de liaison du ligand ICYP aux cellules et la détermination des valeurs IC₅₀ ont été réalisées grâce à l'utilisation d'un programme d'ordinateur adapté aux tests dans le cadre de compétitions simples (12).

Chaque substance de compétition a été testée de 2 à 4 fois dans des expériences indépendantes. Les valeurs Kᵢ ont été calculées à partir des valeurs IC₅₀, tel qu'il est rapporté dans (14).

Les stéréoisomères sont indiqués par (+) ou (-).

Les profils pharmacologiques découlant des possibilités de déplacement du ligand fixé (ICYP) ou non sous l'effet des autres substances se sont avérés être de même nature que ceux qui caractérisent les mêmes récepteurs dans leurs hôtes cellulaires normaux (de mammifères).

Cependant, contrairement à ce que l'on observe dans l'hôte cellulaire normal, aucun couplage ou aucune interaction apparentés n'ont été observés entre le récepteur humain β2-adrénergique et l'adenylate-cyclase en particulier à l'occasion de la mise en contact des ligands actifs avec les cellules transformées, en particulier l'isoproterenol, et ce bien que E.coli contienne le facteur d'élongation Tu, qui est lui-même une protéine G capable de contrôler des synthèses de protéines (17) et d'agir en tant que régulateur de l'activité adenylate-cyclase. Ce manque apparent de couplage est peut être à mettre au compte de différences entre les protéines G de bactéries et les protéines G de mammifères.

Une souche de culture cellulaire de E.coli transformée par un vecteur modifié selon l'invention par le gène hu-β2-adrénergique et désignée par HU-β2-Coli a été déposée à la Collection Nationale de Cultures de Micro-organismes de l'Institut de Paris (CNCM) sous le numéro I-737 le 3 mars 1988.

**Tableau 1**

| composés | propriétés pharmacologiques | Kᵢ (nM) |
|---|---|---|
| (-) norepinephrine | agonist | 9100 ± 1100 |
| (-) epinephrine | agonist | 4700 ± 1400 |
| (+) isoproterenol | agonist | 1400 ± 100 |
| (-) isoproterenol | agonist | 110 ± 10 |
| procaterol | agonist β2 | 40 ± 8 |
| CGP 20712-A | antagonist β1 | 2500 ± 600 |
| practolol | antagonist β1 | 790 ± 220 |
| CGP 12.177 | antagonist | 4.8 ± 1.3 |

### Exemple 2

### Expression dans E.coli des récepteurs β1 et β2 adrénergiques humains.

L'expression dans E.coli des récepteurs β1 et β2 adrénergiques humains (désignés par Huβ1AR et Huβ2AR) en utilisant en tant que vecteur un plasmide dans lequel la région codante des gènes des récepteurs est fusionnée en phase, avec l'extrémité 5′ du gène LamB (23). Des clones stables de bactéries transformées produisant des récepteurs pharmacologiquement actifs ont été obtenus. le profil pharmacologique de chaque récepteur exprimé dans E.coli est analogue à celui observé dans les tissus de mammifères de référence. Ce nouveau système d'expression constitue un mode de réalisation particulièrement avantageux de l'invention. Des clones bactériens exprimant un type de récepteur humain β-adrénergique constituent donc un outil approprié pour le criblage rapide de molécules synthétiques nouvelles.

### Procédure expérimentale

### Souches bactériennes et plasmides

Les souches de E.coli JM101 (25) et pop6510 (24) constituent respectivement les souches réceptrices pour la transformation avec des plamides dérivés de Puc19 (25) et AJC-264 (24).

Le clonage et le séquençage du gène Huβ2AR ont été décrits antérieurement (3). Le gène Huβ1AR a été sélectionné à partir d'une banque de données placentaire humaine dans Emb14, en utilisant comme sonde la région codante du gène du récepteur β-adrénergique de dindon décrite par Yarden et al (Proc. Natl. Acad. Sci 83 p 6795-6799, 1986). La carte de restriction du clone Huβ1AR a d'abord été sous-clonée au site EcoRI de Puc19 décrit dans (12). Un site de restriction NcoI naturellement présent dans le gène Huβ2AR a été crée au niveau du premier codon dans le gène de Huβ1AR. Cette étape a été réalisée en supprimant la région codante 5′ et en la remplaçant avec un oligonucléotide synthétique reconstituant une séquence codon équivalente.

Les plasmides pSMLβ1 et pSMLβ2 portant les gènes hybrides LamB-HuβAR sont dérivés de plasmides multicopies AJC-264 (24). Le plasmide AJC-264 contient le gène LamB sous le contrôle du promoteur ptac12 (23). LamB code pour une protéeine de la membrane externe de E.coli, impliquée dans la perméabilité au maltose et au maltotriose. Ce plasmide contient également une portion de l,lKb comprenant le gène lacI^{Q} et son promoteur. L'expression de LamB est ainsi réprimée à un niveau de base de 1500 monomères. Par induction avec de l'IPTG (10³M) un accroissement de 30 à 40 fois de l'expression de LamB est obtenu (23).

Le plasmide AJC-264 a été coupé par NcoI et StuI : le site NcoI correspond à l'acide aminé 279 de la protéine LamB mature et le site StuI est situé à une distance de 82 paires de bases suivant le dernier codon de LamB. Le fragment NcoI-HindIII de Huβ2AR et le fragment NcoI-Bg12 de Huβ1AR ont été sous-clonés dans les sites NcoI-StuI de pAJC-264. Dans cette construction le même cadre de lecture a été maintenu entre la protéine LamB et les HuβARs. La purification des fragments d'ADN a été réalisée par migration sur de l'agarose 0,8% à bas point de fusion (LMP Agarose Pharmacia) ; les ligations ont été réalisées directement sur le gel en suivant la procédure décrite dans (26). La souche E.coli 6510 a été transformée avec les plasmides recombinants conformément aux techniques standards (26).

### Milieux et conditions de cultures

Les souches recombinantes et les souches de contrôle E.coli ont été cultivées sur un milieu Luria (L-broth) (1% tryptone, 1% NaCl, 0,5% d'extrait de levure, pH ajusté à 7) ou sur un milieu solide L-agar (L-broth solidifié avec 1,5% de bactoagar,DIFCO) contenant 100 µg/ml d'un sel de sodium d'ampicilline (Sigma, St-Louis-USA).

Pour des préparations standard, 50ml de milieu (broth) ont été inoculés avec une colonie unique cultivée une nuit sur des plaques de L-agar solide et incubée à 37°C avec une aération forte. Lorsque la densité optique mesurée à 600nm a atteint 0,4-0,6, de l'IPTG en concentration finale 0,1mM (Sigma, St.Louis-USA) a été ajouté et les cultures ont été placées à 23°C pendant 4 heures. Pour réaliser une préparation plus importante jusqu'à 1 litre, la même procédure générale a été suivie, excepté que le milieu a été inoculé dans une suspension de bactéries en phase exponentielle de croissance, de façon à commencer la culture à 0,05 OD₆₀₀ₘₙ. Les bactéries ont finalement été centrifugées à 1500 g, resuspendues dans un tampon contenant NaCl 100mM, Tris 10mM à pH 7,4 et conservées à 4°C.

### Essais de liaison

Des aliquots contenant 6 x 10⁷ cellules ont été incubés pendant 1 heure à 37°C dans un volume final de 1 ml de ICYP ([¹²⁵]iodocyanopindolol (2080 Ci/mmol, Amersham, UK) à différentes concentrations (de 1 à 80pM). Les réactions ont été arrêtées par filtration sur les filtres Whatman GF-F par 4 lavages rapides avec 4ml de tampon. Le nombre de liaisons non spécifiques déterminé en présence de 1µM de propanolol (-) était inférieur à 25%. Aucune liaison spécifique et saturable n'a pu être mesurée sur le témoin E.coli transfecté avec le plasmide AJC-264. Des expériences de liaison par compétition ont été réalisées de la même façon que dans les essais de liaison directe en utilisant 10 pM de ICYP en tant que ligand marqué radioactivement et des concentrations variées de réactifs de compétition. Chaque médicament inhibait totalement la liaison de l'ICYP au récepteur. Un programme d'ordinateur relatif à la compétition simple a été utilisé pour l'analyse des courbes pour la détermination des valeurs IC₅₀. Les valeurs de K₁ ont été calculées à partir des valeurs de IC₅₀ tel que décrit dans (12).

### Marquage par photoaffinité

Le marquage par photoaffinité a été réalisé sur une suspension de cellules dans 10mM Tris, à pH 7,5 avec 100mM NaCl. Chaque mélange de réaction de 1 ml, contenant 500pM de [¹²⁵I]iodocyanopindolol-diazirine (CYPD, Amersham 1800 Ci/mmole) et environ 100-200pM sites capables de se lier à l'ICYP, a été incubé pendant 1 heure à 37°C en l'absence et en présence de 5µM de (-) propanolol. 5ml de 10mM Tris à pH 7,5 ont été ajoutés et les cellules ont été centrifugées à 5.000xg pendant 10mn. Après une suspension dans 10ml du même tampon, une photolyse a été réalisée et les cellules ont été lavées selon la méthode décrite dans (15). Des bactéries marquées ont été solubilisées dans un échantillon de tampon Laemmli contenant 10% de NaDodSO₄, incubées pendant 2 heures à température ambiante, soumises à une électrophorèse sur gel de polyacrylamide 10% en présence de NaDodSO₄ (16) et autoradiographiées.

Le tableau 2 correspond à la comparaison des K₁ mesurées avec les ligands β-adrénergiques sur Huβ1AR, Huβ2AR et les tissus de mammifères de référence.

### Résultats

Les régions codantes de Huβ1AR et HUβ2AR ont été fusionnées avec la partie 5′ du gène LamB (codant pour les 279 premiers acides aminés de la protéine LamB) portant plusieurs copies du plasmide AJC-264 (24) sous le contrôle du promoteur ptac 12. Des souches E.coli K12,pop 6510 ont été transfectées avec les plasmides résultants, respectivement désignés par pSMLβ1 et pSMLβ2 (fig.4). Des clones stables ont été obtenus,qui présentent une liaison spécifique et saturable pour l'antagoniste β-adrénergique ¹³¹I iodocyanopindolol (ICYP) sous induction avec l'IPTG (fig.5). Comme montré dans les tests de liaison par compétition (fig.5), E.coli transfecté avec pSMLβ1 exprimait les récepteurs β-adrénergiques du type pharmacologique β1 alors que les bactéries contenant pSMLβ2 produisaient les récepteurs β-adrénergiques du sous-type β2. Les récepteurs β1 et β2 adrénergiques présentaient la stéréospécificité attendue, puisque la forme lévogyre du propanolol était liée au récepteur avec une plus grande affinité que la forme dextrogyre.

Environ 220 récepteurs Huβ2AR ont été exprimés par bactérie; le nombre de récepteurs β1 actifs était régulièrement autour de 50 par bactérie ; Ce nombre de récepteurs β1 actifs pourrait être augmenté en diminuant la sensibilité de la protéine exprimée en protéases.

Pour visualiser les deux récepteurs au niveau moléculaire, des expériences de marquage photoaffine ont été réalisées sur les bactéries intactes, en utilisant comme ligand photoactivable, le dérivé diazirine de l'ICYP (fig.6). Les poids moléculaires attendus des protéines de fusion LamB-Huβ1AR et LamB-HUβ2AR sont respectivement de 83 et 72 KDa. Aucune de ces protéines n'est marquée avec le ligand photoactivable. Pour le récepteur β2, la majorité des molécules marquées est sous la forme d'une protéine de 43 KDa ce qui correspond au poids moléculaire théorique du récepteur, clivé de son partenaire de fusion. Comme attendu, un nombre inférieur de récepteurs β1 était marqué par rapport au nombre de récepteurs β2 marqués. Quand l'expérience a été répétée sur un plus grand nombre de bactéries exprimant les récepteurs β1, plusieurs formes moléculaires du récepteur actif ont été trouvées entre environ 40 et 70 KDa, comprenant Huβ1AR libre (poids moléculaire d'environ 47 KDa), des produits de fusion clivés au niveau de l'entité LamB (poids moléculaire d'environ 43 jusqu'à 70 KDa) et une forme de dégradation importante de Huβ1AR (poids moléculaire de 40 KDa). Cette dernière forme correspond probablement au clivage protéolytique de l'entité HUβ1AR au niveau N-terminal ou au niveau C-terminal de la molécule, avec conservation des 7 domaines transmembranaires potentiels, nécessaires pour la constitution d'un récepteur actif. Des expériences de Western blots réalisées sur des préparations membranaires des bactéries exprimant à la fois β1 et β2, avec des anticorps polyclonaux anti-LamB, ont montré que la protéine de fusion non marquée avec le ligand photoactivable constitue en fait le produit immunoréactif majeur.

Le nombre de sites actifs exprimés par les bactéries est suffisant pour réaliser des études pharmacologiques des ligands susceptibles de se lier aux récepteurs β-adrénergiques. Avec 1 litre de culture amené à 1,5 OD₆₀₀ₘₙ, environ 20,000 essais ont été réalisés et les bactéries ayant subi l'induction à l'IPTG ont été conservées dans 15% de glycérol à -70°C pendant une période d'au moins 2 semaines, sans qu'une perte de plus de 20% de leur activité soit constatée. Une série de composés connus pour leur capacité de liaison avec les récepteurs β-adrénergiques de mammifères a été testée dans des expériences de liaison par compétition sur 2 clones bactériens. Comme le montre le tableau 2, la constante K₁ calculée et le rapport K₁β1/K₁β2 sont proches de ceux déterminés dans les tissus de mammifères de référence. On en déduit que l'environnement membranaire de E.coli, qui diffère de celui des cellules de mammifères, n'affecte pas les propriétés particulières de liaison des récepteurs. Les caractéristiques pharmacologiques des récepteurs β-adrénergiques exprimés dans E.coli ainsi que les avantages de la culture bactérienne montrent que les clones bactériens exprimant des récepteurs membranaires constituent des outils particulièrement intéressants pour le criblabe de nouveaux composés synthétiques compte tenu du temps et du coût requis par des procédures mettant en oeuvre des membranes de mammifères enrichies en récepteurs (fig.7).

L'utilisation des vecteurs pSMLβ constitue un moyen préféré pour le niveau d'expression des récepteurs actifs comparé au résultat obtenu dans l'exemple 1 dans lequel Huβ2AR avait été fusionné avec la β-galactosidase. Ceci pourrait être attribué aux propriétés de l'entité LamB, qui constitue une protéine de la membrane externe de E.coli, et facilite l'assemblage correct des récepteurs fusionnés. D'autres polypeptides bactériens de différentes tailles et propriétés pourraient être choisis comme partenaire de fusion pour l'expression des récepteurs transmembranaires. L'observation que le marquage photoaffine de Huβ2AR correspond à la protéeine native clivée du peptide LamB, a permis de transfecter des bactéries avec un plasmide similaire dans lequel le gène Huβ2AR était fusionné à l'extrémité 3′ de la séquence de clivage du peptide signal de la protéine de la membrane interne, de la bactérie.

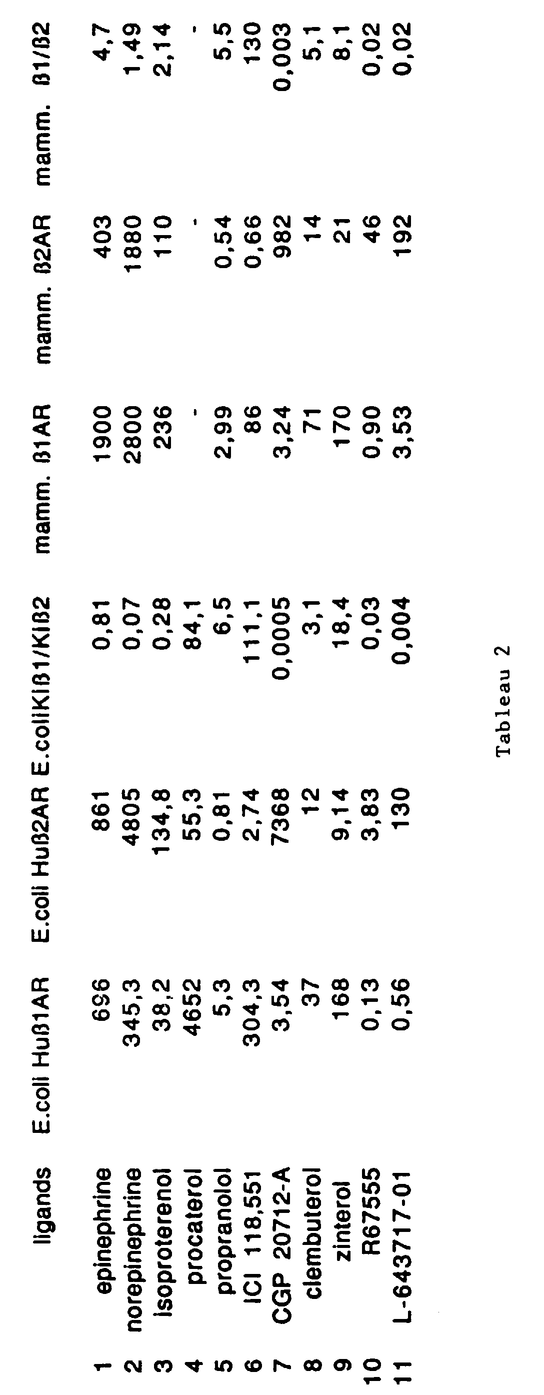

### REFERENCES

1. Frielle, T.Collins, S.Daniel, K.W.Caron, M.G.Lefkowitz, R.J. & Kobilka B.K. Proc.Natl. Acad. Sci. USA. 84.7920-7924(1987).
2. Dixon, R.A.F. et al. Nature 321, 75-79 (1986).
3. Emorine, L.Marullo, S.Delavier-Klutchko, C.Durieu-Trautmann, O. & Strosberg, A.D.Proc. Natl. Acad. Sci. USA. 84.6995-6999 (1987).
4. Kobilka, B.K., Matsui, H. Kobilka, T.S. Yang-Feng, T.L.Francke, U.Caron, M.G.Lefkowitz, R.J.&Regan, J.W. Science 238, 650-656(1987).
5. Kubo, T. et al. Nature 323, 411-416 (1986)
6. Bonner, T.I., Buckley, N.J. Young A.C. & Brann, M.R.Science 237, 527-532 (1987).
7. Masu,Y.Nakayama, K.Tamaki,H.Harada, Y.Kuno, M.& Nakanishi S.Nature 329, 836-838 (1987).
8. Spiegel, A.M.Mol. Cell. Edocrinol. 49, 1-16 (1987)
9. Engelman, D.M.Goldman, A.& Steitz, T.A.Meth.Enzym.88, 81-88 (1982).
10. Huynh, T.V.Young, R.A.& Davis R.W. in DNA Cloning, a Practical Approach Vol.1, 49-78 (IRL,Oxford, 1985)
11. Minneman, K.P.Hegstrand, C.R.& Molinoff, P.B. Mol. Pharmacol. 16,34-46 (1979).
12. Cheng, Y.& Prusoff, W.H.Biochem. Pharmacol.22, 3099-3108 (1973).
13. Fowler, A.V.& Zabin I.J.Biol.Chem.258, 14354-14358 (1983)
14. Rousset, J.P.Gilson, E.& Hofnung M.J.Mol.Biol. 191, 313-320 (1986)
15. Cervantes-Olivier. P.Delavier-Klutchko, C.Durieu-Trautmann, O.Kaveri, S.Desmandril, M.& Strosberg, A.D.Biochem. J.Biochem J 250:133-143.
16. Laemmli, U.K.& Favre M.J.Mol. Biol. 80, 575-599 (1973)
17. Reddy. P. Miller, D.& Peterkofsky, A.J.Biol. Chem. 261, 11448-11451 (1986).
18. Stiles, G.Arch. Biochem. Biophys. 237, 65-71 (1985)
19. Cervantes-Olivier. P. Durieu-Trautmann, O. Delavier-Klutchko, C.& Strosberg, A.D.Biochemistry 24, 3765-3770 (1985).
20. Hanada, K. Yamato, I.& Anraku, Y.J.Biol. Chem. 262, 14100-14104 (1987).
21. Allen M.Spiegel, Signal transduction by guanine nucleotide binding proteins ; Molecular and Cellular Endocrinology, 49(1987)1-16 Elsevier Scientific Publishers Ireland, Ltd.
22. Marullo,S.et al. Proc. Natl. Acad.Sci.USA.85:7551-7555. 1987
23. Boulain,J.C.et al.1986. Mutagenesis by random linker insertion into the lamB gene of Escherichia coli K12.
24. Charbit, A et al. 1986. EMBO J.5:3029-3037
25. Yanisch-Perron,C.et al. 1985-Gene 33.103-119
26. In Current protocols in molecular biology 1989. Ausubel, F.M. et al (Ed) Greene Publishing associates and Wiley-Interscience, NEW YORK.

## Revendications

1. Vecteur réplicable dans une culture de levures contenant un insérat de nucléotides codant pour une séquence d'acides aminés contenue dans une protéine de mammifère et ayant une activité biologique de récepteur membranaire, ce vecteur étant caractérisé en ce que l'insérat est placé sous le contrôle d'une séquence, incluse dans ce vecteur autorisant l'expression de cet insérat dans les levures, et comportant notamment un promoteur reconnu par les polymérases de ces levures, et en ce que cet insérat code pour une séquence d'acides aminés contenue dans une protéine présentant des éléments de structure en commun avec les récepteurs membranaires susdits, notamment les récepteurs α- ou β-adrénergiques, les récepteurs muscariniques, les récepteurs neuropeptidiques, en particulier la "substance K", l'expression de l'insérat s'accompagnant également de l'exposition à l'extérieur de ces levures des sites caractéristiques des récepteurs membranaires qui affleurent à la surface des cellules de mammifères d'origine.

2. Vecteur selon la revendication 1, caractérisé en ce que le promoteur est un promoteur d'alcool-déshydrogénase I ou du gène galactokinase.

3. Vecteur modifié selon la revendication 1 ou la revendication 2, caractérisé en ce que l'insérat contenu dans le vecteur code pour une séquence d'acides aminés contenue dans une protéine eucaryote comportant dans la structure au sein de la membrane plasmique, sept segments transmembranaires hydrophobes entre lesquels s'intercalent des boucles extra et intracellulaires, une région aminoterminale extracellulaire et une région cytoplasmique carboxyterminale, et des sites de liaison constitués par des régions hydrophiles formées dans la membrane.

4. Vecteur selon l'une quelconque des revendications 1 à 3, caractérisé en ce que l'insérat code pour un récepteur humain α-adrénergique.

5. Vecteur selon l'une quelconque des revendications 1 à 3, caractérisé en ce que l'insérat code pour un récepteur humain β-adrénergique.

6. Vecteur selon l'une quelconque des revendications 1 à 3, caractérisé en ce que l'insérat code pour un récepteur muscarinique.

7. Vecteur selon l'une quelconque des revendications 1 à 6, caractérisé en ce que la levure est S. cerevisiae et en ce que le promoteur est celui de l'alcool déshydrogénase I ou du gène galactokinase.

8. Vecteur selon l'une quelconque des revendications 1 à 7, caractérisé en ce que l'insérat est dépourvu de la région promotrice qui lui est normalement associée dans les cellules de mammifère, qui constituent l'hôte normal de la séquence codante contenue dans l'insérat.

9. Levures transformées par un vecteur modifié selon l'une quelconque des revendications 1 à 8, et capables de synthétiser un polypeptide comportant, après induction de l'expression de l'insérat, des séquences affleurant à leur surface et reconnues par les ligands reconnaissant le récepteur membranaire naturel.

10. Culture cellulaire transformée par un vecteur modifié, désignée par HU-β2-Coli et déposée à la CNCM sous le numéro I-737.

11. Procédé de détection de la capacité d'une molécule à se comporter comme ligand vis-à-vis d'un récepteur appartenant à la classe des récepteurs normalement fixés à une protéine G, caractérisé par :
- la mise en contact de la molécule avec un organisme unicellulaire à l'exception des lignées cellulaires de mammifères, préalablement transformé par un vecteur lui-même modifié par un insérat codant pour ce récepteur membranaire, cet organisme exprimant ledit récepteur membranaire, le cas échéant après induction de l'expression de cet insérat, et portant à sa surface un ou plusieurs sites spécifiques de ce récepteur, cette mise en contact étant effectuée dans des conditions permettant la formation d'une liaison entre l'un au moins de ces sites spécifiques et ladite molécule dès lors qu'elle s'avérerait effectivement posséder une affinité pour ce récepteur ;
- la détection de la formation éventuelle d'un complexe du type ligand-récepteur.

12. Procédé pour l'étude de l'affinité d'un récepteur de mammifère appartenant à la classe de ceux qui sont couplés à une protéine G, pour un ou plusieurs ligands déterminés, caractérisé par :
- la transformation d'une culture unicellulaire avec un vecteur, à l'exception des lignées cellulaires de mammifères, notamment un plasmide ou un phage, dans lequel avait auparavant été incorporée une séquence de nucléotides codant pour le polypeptide contenu dans ce récepteur de mammifère, sous le contrôle d'éléments de régulation, notamment d'un promoteur, permettant l'expression dans la culture unicellulaire utilisée, de ladite séquence de nucléotides, et l'exposition à l'extérieur de ces hôtes unicellulaires, des sites caractéristiques des récepteurs membranaires qui affleurent à la surface des cellules de mammifères d'origine,
- la culture des organismes unicellulaires transformés dans des conditions permettant l'expression dudit insérat,
- la mise en contact de ces organismes unicellulaires avec ces ligands déterminés,
- la détection d'une réaction affine entre lesdits organismes unicellulaires transformés et lesdits ligands déterminés.

13. Kit pour la détection de l'affinité éventuelle d'un ligand pour un récepteur membranaire de mammifère déterminé comprenant :
- une culture de levures transformées par un vecteur modifié tel que défini dans l'une quelconque des revendications 1 à 8 ou une culture de levures selon la revendication 9,
- des moyens physiques ou chimiques pour induire l'expression de la séquence nucléotidique contenue dans le vecteur modifié lorsque le promoteur placé en amont de cette séquence est un promoteur inducible par lesdits moyens physiques ou chimiques,
- un ou plusieurs ligands témoins ayant des affinités déterminées pour ledit récepteur membranaire,
- des moyens physiques ou chimiques pour la caractérisation de l'activité biologique de ladite protéine.

14. Méthode pour l'expression d'un récepteur mammifère à la surface des cellules de bactéries ou de levures, caractérisée par :
- la transformation d'une culture de bactéries ou de levures avec un vecteur, ce vecteur contenant un insérat placé sous le contrôle d'une séquence, incluse dans ce vecteur autorisant l'expression de cet insérat dans les bactéries ou les levures, et comportant notamment un promoteur reconnu par les polymérases de ces bactéries ou de ces levures, et en ce que cet insérat code pour une séquence d'acides aminés contenue dans une protéine présentant des éléments de structure en commun avec les récepteurs membranaires susdits, notamment les récepteurs α- ou β-adrénergiques, les récepteurs muscariniques, les récepteurs neuropeptidiques, en particulier la "substance K", l'expression de l'insérat s'accompagnant également de l'exposition à l'extérieur de ces bactéries ou de ces levures des sites caractéristiques des récepteurs membranaires qui affleurent à la surface des cellules de mammifères d'origine, ledit vecteur étant choisi notamment parmi les plasmides ou les phages, et la culture de bactéries ou de levures étant transformée dans des conditions permettant l'expression dudit insérat.

## Patentansprüche

1. Vektor, der in einer Hefekultur replizierbar ist, enthaltend eine Nucleotidinsertion, die eine Aminosäuresequenz codiert, die in einem Säugerprotein enthalten ist und eine biologische Aktivität eines Membranrezeptors hat, wobei dieser Vektor dadurch gekennzeichnet ist, daß die Insertion unter die Kontrolle einer in dem Vektor enthaltene Sequenz gestallt ist, die die Expression dieser Insertion in den Hefen ermöglicht, und die insbesondere einen Promotor aufweist, der durch die Polymerasen dieser Hefen erkannt wird, und dadurch, daß diese Insertion eine Aminosäuresequenz codiert, die in einem Protein enthalten ist, das mit den obengenannten Membranrezeptoren gemeinsame Strukturelemente aufweist, insbesondere den α- oder β-adrenergen Rezeptoren, den muskarinischen Rezeptoren, den Neuropeptidrezeptoren, insbesondere der "Substanz K", wobei die Expression der Insertion auch verbunden ist mit der Exposition charakteristischer Bereiche von Membranrezeptoren, die an der Oberfläche von Zellen aus Säugern vorhanden sind, auf der Außenseite dieser Hefen.

2. Vektor nach Anspruch 1, dadurch gekennzeichnet, daß der Promotor ein Promotor der Alkoholdehydrogenase I oder des Galaktokinasegens ist.

3. Modifizierter Vektor nach Anspruch 1 oder Anspruch 2, dadurch gekennzeichnet, daß die in dem Vektor enthaltene Insertion eine Aminosäuresequenz codiert, die in einem eukaryontischen Protein enthalten ist, das in der Struktur, die innerhalb der Plasmamembran liegt, sieben hydrophobe Transmembransegmente aufweist, zwischen denen extra- und intrazelluläre Schleifen liegen, eine extrazelluläre aminoterminale Region und eine cytoplasmatische carboxyterminale Region und Bindungsstellen, die durch hydrophile Regionen, die sich in der Membran ausbilden, gebildet werden.

4. Vektor nach einem der Ansprüche 1 bis 3, dadurch gekennzeichnet, daß die insertion einen menschlichen α-adrenergen Rezeptor codiert.

5. Vektor nach einem der Ansprüche 1 bis 3, dadurch gekennzeichnet, daß die Insertion einen menschlichen β-adrenergen Rezeptor codiert.

6. Vektor nach einem der Ansprüche 1 bis 3, dadurch gekennzeichnet, daß die Insertion einen muskarinischen Rezeptor codiert.

7. Vektor nach einem der Ansprüche 1 bis 6, dadurch gekennzeichnet, daß die Hefe S. cerevisiae ist, und dadurch, daß der Promotor der der Alkoholdehydrogenase I oder des Galaktokinasegens ist.

8. Vektor nach einem der Ansprüche 1 bis 7, dadurch gekennzeichnet, daß der Insertion die Promotorregion fehlt, die normalerweise mit ihr verbunden ist in den Säugerzellen, die den normalen Wirt der in der Insertion enthaltenen codierenden Sequenz darstellen.

9. Hefen, die mit einem modifizierten Vektor nach einem der Ansprüche 1 bis 8 transformiert sind und die in der Lage sind, ein Polypeptid zu synthetisieren, und die nach der Induktion der Expression der Insertion Sequenzen aufweisen, die an ihrer Oberfläche auftreten und die von Liganden erkannt werden, die den natürlichen Membranrezeptor erkennen.

10. Zellkultur, die mit einem modifizierten Vektor transformiert ist, mit der Bezeichnung HU-β2-Coli und hinterlegt bei CNCM unter der Nummer I-737.

11. Verfahren zum Nachweis der Fähigkeit eines Moleküls, sich wie ein Ligand zu einem Rezeptor zu verhalten, der der an G-Proteine gebundenen Rezeptorklasse angehört, gekennzeichnet durch:
- das Inkontaktbringen des Moleküls mit einem einzelligen Organismus, ausgenommen Säugerzellinien, der zuvor mit einem Vektor transformiert wurde, der seinerseits durch eine Insertion modifiziert wurde, die diesen Membranrezeptor codiert, wobei dieser Organismus diesen Membranrezeptor exprimiert, gegebenenfalls nach Induktion der Expression dieser Insertion, und auf seiner Oberfläche einen oder mehrere spezifische Bereiche dieses Rezeptors aufweist und wobei dieses Inkontaktbringen unter Bedingungen durchgeführt wird, die die Ausbildung einer Bindung zwischen mindestens einem dieser spezifischen Bereiche und dem Molekül erlauben, sobald es eine wirksame Affinität zu diesem Rezeptor aufweist;
- den Nachweis eines etwaigen Komplexes vom Typ Ligand-Rezeptor.

12. Verfahren zur Untersuchung der Affinität eines Rezeptors aus Säugern, der der an ein G-Protein gekoppelten Klasse angehört, zu einem oder mehreren ausgesuchten Liganden, gekennzeichnet durch:
- die Transformation einer Einzellerkultur, wobei Säugerzellinien ausgenommen sind, mit einem Vektor, insbesondere einem Plasmid oder einem Phagen, in den vorher eine Nucleotidsequenz eingefügt wurde, die das in diesem Säugerrezeptor enthaltene Polypeptid codiert, unter der Kontrolle regulatorischer Elemente, insbesondere eines Promotors, die die Expression der Nucleotidsequenz in der verwendeten Einzellerkultur erlauben; und die Exposition charakteristischer Bereiche von Membranrezeptoren, die auf der Oberfläche von Säugerzellen auftreten, auf der Außenseite dieser einzelligen Wirte;
- die Kultivierung der transformierten einzelligen Organismen unter Bedingungen, die die Expression der Insertion erlauben;
- das Inkontaktbringen dieser einzelligen Organismen mit den ausgesuchten Liganden;
- den Nachweis einer Bindungereaktion zwischen den transformierten einzelligen Organismen und den ausgesuchten Liganden.

13. Kit zum Nachweis der etwaigen Affinität eines Liganden zu einem bestimmten Membranrezeptor aus Säugern umfassend:
- eine Kultur von Hefen, die mit einem modifizierten Vektor wie in einem der Ansprüche 1 bis 8 definiert transformiert sind, oder eine Kultur von Hefen nach Anspruch 9;
- physikalische oder chemische Mittel zur Induktion der Expression der Nucleotidsequenz, die in dem modifizierten Vektor enthalten ist, wenn der stromaufwärts dieser Sequenz plazierte Promotor ein Promotor ist, der durch die physikalischen oder chemischen Mittel induzierbar ist;
- einen oder mehrere Kontrolliganden, die bekannte Affinitäten zu dem Membranrezeptor haben,
- physikalische oder chemische Mittel zur Charakterisierung der biologischen Aktivität des Proteins.

14. Verfahren zur Expression eines Rezeptors aus Säugern an der Oberfläche von Bakterien- oder Hefezellen gekennzeichnet durch:
- die Transformation einer Bakterien- oder Hefekultur mit einem Vektor, wobei dieser Vektor eine Insertion enthält, die unter die Kontrolle einer in dem Vektor enthaltenen Sequenz gestellt ist, die die Expression dieser Insertion in den Bakterien oder Hefen gewährleistet, und die insbesondere einen Promotor aufweist, der von den Polymerasen dieser Bakterien oder dieser Hefen erkannt wird, und dadurch, daß diese Insertion eine Aminosäuresequenz codiert, die in einem Protein enthalten ist, das gemeinsame Strukturelemente mit den obengenannten Membranrezeptoren aufweist, insbesondere den α- oder β-adrenergen Rezeptoren, den muskarinischen Rezeptoren, den Neuropeptidrezeptoren, insbesondere der "Substanz K", und die Expression der Insertion auch verbunden ist mit der Exposition charakteristischer Bereiche von Membranrezeptoren, die an der Oberfläche von Zellen aus Säugern auftreten, an der Außenseite dieser Bakterien oder Hefen, wobei der Vektor insbesondere ausgewählt ist aus Plasmiden oder Phagen;
- die Kultivierung der transformierten Bakterien oder Hefen unter Bedingungen, die die Expression der Insertion erlauben.

## Claims

1. Vector which can be replicated in a yeast culture containing an insertion of nucleotides coding for an amino acid sequence contained in a mammalian protein and having a membrane-receptor biological activity, this vector being characterized in that the insertion is placed under the control of a sequence, included in this vector, which authorizes the expression of this insertion in the yeasts, and comprising in particular a promoter which is recognized by the polymerases of these yeasts, and in that this insertion codes for an amino acid sequence contained in a protein having structural elements in common with the abovesaid membrane receptors, in particular α- or β- adrenergic receptors, muscarinic receptors, neuropeptide receptors, in particular the "substance K" receptor, the expression of the insertion also being accompanied by the exposure, to the outside of these yeasts, of sites characteristic of membrane receptors which lie on the surface of cells of mammalian origin.

2. Vector according to Claim 1, characterized in that the promoter is a promoter of alcohol dehydrogenase I or of the galactokinase gene.

3. Modified vector according to Claim 1 or Claim 2, characterized in that the insertion contained in the vector codes for an amino acid sequence contained in a eukaryotic protein including in the structure, within the plasma membrane, seven hydrophobic transmembrane segments between which are intercalated extra- and intercellular loops, an extracelluar amino-terminal region and a cytoplasmic carboxy-terminal region, and binding sites consisting of hydrophilic regions formed in the membrane.

4. Vector according to any one of Claims 1 to 3, characterized in that the insertion codes for a human α-adrenergic receptor.

5. Vector according to any one of Claims 1 to 3, characterized in that the insertion codes for a human β-adrenergic receptor.

6. Vector according to any one of Claims 1 to 3, characterized in that the insertion codes for a muscarinic receptor.

7. Vector according to any one of Claims 1 to 6, characterized in that the yeast is S. cerevisiae and in that the promoter is that of alcohol dehydrogenase I or of the galactokinase gene.

8. Vector according to any one of Claims 1 to 7, characterized in that the insertion lacks the promoter region which is normally associated with it in mammalian cells, which constitutes the normal host of the coding sequence contained in the insertion.

9. Yeasts transformed by a modified vector according to any one of Claims 1 to 8, and capable of synthesizing a polypeptide containing, after induction of expression of the insertion, sequences lying on their surface and recognized by the ligands which recognize the natural membrane receptor.

10. Cell culture transformed by a modified vector, denoted by HU-β2-Coli and filed at the CNCM under the number I-737.

11. Process for detection of the capacity of a molecule to behave as ligand towards a receptor belonging to the class of receptors normally bound to a G protein, characterized by:
- placing the molecule in contact with a unicellular organism, with the exception of mammalian cell lines, which has been previously transformed by a vector which is itself modified by an insert coding for this membrane receptor, this organism expressing the said membrane receptor, if necessary after induction of expression of this insert and bearing at its surface one or more specific sites for this receptor, this placing in contact being carried out under conditions which allow the formation of a bond between at least one of these specific sites and the said molecule as soon as it has effectively proven itself to have an affinity for this receptor;
- detection of any possible formation of a complex of the ligand-receptor type.

12. Process for studying the affinity of a mammalian receptor belonging to the class of those which are coupled to a G protein, for one or more determined ligands, characterized by:
- the transformation of a unicellular culture with a vector, with the exception of mammalian cell lines, in particular a plasmid or a phage, into which has previously been incorporated a nucleotide sequence coding for the polypeptide contained in this mammalian receptor, under the control of regulation elements, in particular a promoter, which allows the expression, in the unicellular culture used, of the said nucleotide sequence, and the exposure, to the outside of these unicellular hosts, of sites characteristic of membrane receptors which lie on the surface of cells of mammalian origin.
- the culturing of the unicellular organisms transformed under conditions which allow the expression of the said insertion,
- the placing in contact of these unicellular organisms with these determined ligands,
- the detection of an affinity reaction between the said transformed unicellular organisms and the said determined ligands.

13. Kit for detecting any possible affinity of a ligand for a determined mammalian membrane receptor, comprising:
- a culture of yeasts transformed by a modified vector as defined in any one of Claims 1 to 8 or a yeast culture according to Claim 9,
- physical or chemical means for inducing the expression of the nucleotide sequence contained in the modified vector when the promoter placed upstream of this sequence is a promoter which can be induced by the said physical or chemical means,
- one or more control ligands which have determined affinities for the said membrane receptor,
- physical or chemical means for characterizing the biological activity of the said protein.

14. Method for expressing a mammalian receptor on the surface of bacterial cells or of yeast cells, characterized by:
- the transformation of a bacterial culture or a yeast culture with a vector, this vector containing an insertion placed under the control of a sequence, included in this vector, which authorizes the expression of this insertion in the bacteria or the yeasts, and comprising in particular a promoter which is recognized by the polymerases of these bacteria or of these yeasts, and in that this insertion codes for an amino acid sequence contained in a protein having structural elements in common with the abovesaid membrane receptors, in particular α- or β-adrenergic receptors, muscarinic receptors, neuropeptide receptors, in particular the "substance K" receptor, the expression of the insertion also being accompanied by exposure, to the outside of these bacteria or of these yeasts, of sites characteristic of membrane receptors which lie on the surface of cells of mammalian origin, the said vector being chosen in particular from plasmids or phages, and the bacterial culture or the yeast culture being transformed under conditions which allow the expression of the said insertion.
